# EUROPÄISCHE PATENTANMELDUNG

(11) **EP 2 554 651 A1**
(43) Veröffentlichungstag der Anmeldung: **06.02.2013**
(21) Anmeldenummer: 11006447.4
(22) Anmeldetag: 05.08.2011
(51) Int. Cl.: C12M 1/107

(54) **Verfahren zum Betreiben einer Biogasanlage und Biogasanlage zur Durchführung dieses Verfahrens**

(71) Anmelder: Holm, Niels Christian, 32425 Minden (DE)
(72) Erfinder: Holm, Niels Christian, 32425 Minden (DE)
(74) Vertreter: Tönnies, Jan G.

(57) **Zusammenfassung**

Verfahren zum Betreiben einer Biogasanlage, bei dem der anfallende Rohgärrest unter Gewinnung eines flüssigen Eindampfkonzentrats entwässert und getrocknet wird, bei dem das flüssige Eindampfkonzentrat zu dem Rohgärrest vor oder nach der Entwässerung rückgemischt wird.

## Beschreibung

Die Erfindung betrifft ein Verfahren zum Betreiben einer Biogasanlage unter Elimination von Eindampf - Konzentraten aus der Gärrestbehandlung und eine Biogasanlage zur Durchführung dieses Verfahrens.

Unter den erneuerbaren Energien nimmt die Biogastechnologie eine Schlüsselstellung ein, weil sie nicht nur klimaneutral Energie liefert, sondern auch ein bedeutender Baustein bei der Behandlung / Entgiftung / oder Entsorgung organischer Abfälle / Reststoffe sein kann und weil die Biogasproduktion flexibel mit den Produktionsschwankungen anderer erneuerbarer Energien (insbesondere Wind und Sonne) kombiniert werden kann.

Des Weiteren liefert die Biogastechnologie mit den Gärresten nach der Vergärung ein Produkt, dessen Qualität als organischer Biodünger sehr oft die entsprechenden Qualitäten der Input-Substrate übersteigt und diese meist erst zu Biodünger macht.

Allerdings fallen sehr oft sehr große Mengen an Gärresten mit nur sehr geringen Nährsroffkonzentrationen (im Wesentlichen sind hiermit Stickstoff = N, Phosphor = P und Kalium = K gemeint) an. Die Speicherung und der Transport dieser Gärreste sind mit sehr hohen Kosten verbunden. Das gilt insbesondere für Gegenden, wo eine landwirtschaftliche Überversorgung mit Nährstoffen besteht, beispielsweise durch ein Übermaß an produzierter Gülle aus der Intensivtierhaltung. Hiervon sind in Europa insbesondere Niedersachsen, Holland, Belgien, Norditalien und Dänemark betroffen. Aus diesen Gegenden wird in großem Maßstab Gülle exportiert, weil vor Ort die Flächen fehlen um die Gülle aus zu bringen. Das führt dazu, dass in diesen Gegenden produzierte Gärreste oft übermäßig weit transportiert werden müssen.

Daher nimmt die Bedeutung der Gärrestbehandlung, bei der es sich nahezu immer um eine Mengenreduktion der Gärreste handelt, stetig zu.

Zu den wirtschaftlich bedeutenden Gärrestbehandlungsverfahren gehören unterschiedliche Entwässerungsverfahren und das Gärresttrocknungsverfahren. Um die großen Volumina der Flüssigfraktionen aus den Gärrestentwässerungen zu verringern, werden die unterschiedlichsten biologischen Verfahren und Membranverfahren eingesetzt. Neuerdings ist auch der Einsatz der Eindampfung der Flüssigfraktionen hinzugekommen.

Die Eindampfung bietet den großen Vorteil der vollständigen Rückgewinnung aller Nährstoffe in konzentrierter, flüssiger Form sowie die Generierung eines sehr unbelasteten Kondensates, das entweder direkt oder nach einer wenig intensiven biologischen Nachbehandlung direkt in einen Vorfluter abgeleitet werden kann. Die Konzentratmenge ist hierbei immer wesentlich geringer als die Kondensatmenge.

Die Verfahrenskombination Entwässerung (einstufig oder mehrstufig) mit nachfolgender Eindampfung (sowie gegebenenfalls nachfolgender Trocknung) der Flüssigfraktion der Entwässerung beinhaltet allerdings den Nachteil, dass zwei verschiedene konzentrierte Gärrestfraktionen generiert werden. Zudem ist der Wasseranteil der Eindampfkonzentrate immer noch sehr hoch (üblicherweise 75 - 95 %). Das bedeutet, dass bei Einsatz von Inputsubstraten mit nicht hohem Biogaspotential (wie z.B. Gülle, Kot, Mist sowie anderen organischen Reststoffen) der Wärme - Energiegehalt des produzierten Biogases (überwiegend in Form der Abwärme der Blockheizkraftwerke) oft nicht ausreichend ist, um die verschiedenen Fraktionen (die entwässerten Gärreste und das Eindampfkonzentrat) vollständig zu trocknen.

Der Erfindung liegt damit die Aufgabe zugrunde, ein Verfahren und eine Anlage zur Durchführung dieses Verfahrens zu schaffen, die zu einer Wasserverringerung und damit zu einer Mengenverringerung aller Gärrestkonzentrate bei reduziertem Wärmebedarf führt.

Erfindungsgemäß wird diese Aufgabe durch die Merkmale der Ansprüche 1 bzw. 4 gelöst. Die Unteransprüche 2 und 3 geben vorteilhafte Ausführungsformen wieder.

Vor der Erläuterung des neuen Verfahrens werden die hierfür wichtigen, grundlegenden Eigenschaften der beiden Konzentrate eingeführt:
Die entwässerten und eingedampften Gärreste bestehen aus Wasser und aus dem so genannten durch Verdampfung des Wassers im Labor ermittelten Trockenrückstand (= TR). Dieser Trockenrückstand besteht aus einem partikulärem Anteil TRP (überwiegend Faulschlammflocken) sowie aus einem ursprünglich in der Wasserfraktion gelösten Anteil TRI (überwiegend wasserlösliche Ionen wie Kalium, Magnesium, Kalzium, Phosphat, Karbonat, Chlorid usw.). Es gilt: TR = TRP + TRI.

Der TR in der entwässerten Gärrestfraktion besteht überwiegend aus TRP und der TR im Eindampfkonzentrat überwiegend aus TRI.

Erfindungsgemäß wird die Mengenverringerung erreicht, indem die Wasserfraktion der entwässerten Gärreste (die eine viel geringe TRI Konzentration aufweist als die flüssigen Eindampfkonzentrate) teilweise durch die flüssigen Eindampfkonzentrate "ersetzt" wird, die flüssigen Eindampfkonzentrate somit vollständig oder teilweise Teil der Wasserfraktion der entwässerten Gärreste werden. Das wird erreicht, indem die flüssigen Eindampfkonzentrate in die Rohgärreste nach der Entwässerung und/oder vor der Entwässerung rückgemischt werden.

### Beispiel: Rückmischung nach der Entwässerung:

### Bisherige Verfahrensweise:

Da die Input-Substrate von Biogasanlagen (Mais, Gras, Stroh, Ganzpflanzensilase und ähnliches) oft über viel Strukturmaterial verfügen, wird meistens eine 2 - stufige Entwässerung gewählt, wobei in der ersten Stufe Schneckenpressen zum Einsatz kommen, die fast ausschließlich noch vorhandenes Strukturmaterial aus dem Rohgärrest entnehmen und aufkonzentrieren.

Es wird ausgegangen von 720 m³/d Rohgärreste nach der Vergärung mit einem TRP von 10 % und einem TRI von 1 %. Bei der Entwässerung wird nur der strukturelle TRP aus dem Rohgärrest entfernt und dieser auf 18 % TRP entwässert und ca. 6 % TRP in der Flüssigfraktion verbleibt. Die Bilanz dieser ersten Entwässerungsstufe ergibt somit:

| | |
|---|---|
| Rohgärrest: | 720 m³/d, 10 % TRP, 1 % TRI, 89 % Wasser |
| Entwässerter Gärrest: | 240 t/d, 18 % TRP, 1 % TRI, 81 % Wasser |
| Flüssigfraktion: | 480 m³/d, 6 % TRP, 1 % TRI, 93 % Wasser |

Für die zweite Entwässerungsstufe (z.B. mit Zentrifugen) wird der Einfachheit halber davon ausgegangen, dass die Entwässerung den TRP vollständig aus dem Rohgärrest entfernt und diesen auf 24 % TRP entwässert. (In der betrieblichen Praxis erfolgt die Entfernung der restlichen TRP Fraktionen aus den Flüssigfraktionen der Entwässerung meistens mittels einer nachfolgenden Hochlastbiologie; das ist aber für die vorliegenden Zwecke und Erläuterungen unerheblich und wird daher vernachlässigt).

Die Bilanz der zweiten Entwässerungsstufe ergibt somit:

| | |
|---|---|
| Rohgärrest: | 480 m³/d, 6 % TRP, 1 % TRI, 93 % Wasser |
| Entwässerter Gärrest: | 120 dd, 24 % TRP, 1 % TRI, 75 % Wasser |
| Flüssigfraktion: | 360 m³/d, 0 % TRP, 1 % TRI, 99 % Wasser |

Bei der Eindampfung wird von einer Aufkonzentrierung auf 5 % TRI ausgegangen. Bei der Bilanzierung werden die NH₄/NH₃ Konzentrationen nicht beachtet, weil diese im Rahmen der Eindampfung üblicherweise mittels Strippung eliminiert bzw zurückgewonnen werden.

Die Bilanz der Eindampfung ergibt somit:

| | |
|---|---|
| Flüssigfraktion aus der Entwässerung: | 360 m³/d, 0 % TRP, 1 % TRI, 99 % Wasser |
| Eindampfkonzentrat: | 72 m³/d, 0 % TRP, 5 % TRI, 95 % Wasser |
| Flüssigfraktion = Kondensat: | 288 m³/d, 0 % TRP, 0 % TRI, 100 % Wasser |

Da bei diesem Beispiel die Rückmischung nach der Entwässerung der ersten Entwässerungsstufe betrachtet, verbleibt in den nachfolgenden Betrachtungen der entwässerte Gärrest der zweiten Entwässerungsstufe unberücksichtigt.

Die beiden Konzentratfraktionen entwässerter Gärrest der ersten Entwässerungsstufe und das Eindampfkonzentrat zusammen ergeben somit 312 t/d mit 13,846 % TRP und 1,923 % TRI (= 15,77 % TR). Der Rest mit 84,23 % = 262,8 m³ ist Wasser.

Die Bilanz der Trocknung dieser beiden Fraktionen auf 90 % TR ergibt somit:

| | |
|---|---|
| Konzentrate: | 312 m³/d, 15,77 % TR, 84,23 % Wasser |
| Trockengranulat: | 54,67 t/d, 90 % TR, 10 % Wasser |
| Verdampftes Wasser: | 257,33 m³/d, 0 % TR |

Nach diesem herkömmlichen Verfahren müssen somit bei vollständiger Gärresttrocknung dieser beiden Fraktionen 257,33 m³/d = 10,72 m³/h Wasser verdampft werden. Mit einer Trocknungseffektivität von 1 MW_{Wärme}/m³ Wasser besteht somit ein Wärmebedarf von 10,72 MW.

Für die Einbringung der flüssigen Eindampfkonzentrate in die Trocknung muss mit Trockengranulat rückgemischt werden; das beinhaltet erhebliche finanzielle Mehraufwendungen.

Die einzelnen Behandlungsstufen müssen daher wie folgt ausgelegt werden:

| Durchsatz hydraulisch/d / Durchsatz TRP/d Verdampfungsleistung | | | |
|---|---|---|---|
| 1. Entwässerung | 720 m³/d | 72 t/d | |
| Eindampfung | 360 m³/d | | 12,00 m³/h |
| Trocknung | 312 t/d | | 10,72 m³/h |

### Verfahren nach Anspruch 2:

Von den 240 t/d entwässerter Gärrest der ersten Entwässerungsstufe mit 18 % TRP und 1 % TRI werden 180 t/d mit genau 180 m³/d Eindampfkonzentrat, 5 % TRI, auf dann genau 9 % TRP und genau 3 % TR vermischt.

Diese 360 m³/d werden erneut mit einer Schneckenpresse auf 180 t/d 18 % TRP entwässert (der Einfachheit halber wird davon ausgegangen, dass die Flüssigfraktion 0 % TRP enthält). Genau dann bleibt kein Eindampfkonzentrat übrig. Die Bilanz dieser zusätzlichen Entwässerung ergibt somit:

| | |
|---|---|
| Entw. Gärrest + Eindampfkonz.: | 360 m³/d, 9 % TRP, 3 % TRI, 88 % Wasser |
| Entwässerter Gärrest: | 180 t/d, 18 % TRP, 3 % TRI, 79 % Wasser |
| Flüssigfraktion: | 180 m³/d, 0 % TRP, 3 % TRI, 97 % Wasser |

Bei der Eindampfung wird wiederum von einer Aufkonzentrierung auf 5 % TRI ausgegangen. Da der Eindampfung die zusätzlichen 180 m³/d zugeführt werden, ergibt die Bilanz der Eindampfung somit:

| | | | | |
|---|---|---|---|---|
| Flüssigfraktion aus den Entwässerungen: | 540 m³/d, 0 % TRP, | 1,667 % TRI, | 98,33 | % Wasser |
| Eindampfkonzentrat: | 180 m³/d, 0 % TRP, | 5 % TRI, | 95 | % Wasser |
| Flüssigfraktion = Kondensat: | 360 m³/d, 0 % TRP, | 0 % TRI, | 100 | % Wasser |

Da die 180 m³/d Eindampfkonzentrat vollständig rezirkuliert werden müssen (siehe oben), damit der ganze TRI in der Wasserphase der entwässerten Gärreste der ersten Entwässerungsstufe landet, verbleibt keinerlei zu speicherndes und zu entsorgendes Eindampfkonzentrat.

Somit müssen nur die beiden entwässerten Gärrestfraktionen (180 t/d mit 3 % TRI und 60 t/d mit 1 % TRI, beide 18 % TRP) auf 90 % TR getrocknet werden:

| | |
|---|---|
| Entwässerte Gärreste: | 240 m³/d, 20,5 % TR, 79,5 % Wasser |
| Trockengranulat: | 54,67 t/d, 90 % TR, 10 % Wasser |
| Verdampftes Wasser: | 185,33 m³/d, 0 % TR |

Nach diesem erfindungsgemäßen Verfahren müssen somit bei vollständiger Gärresttrocknung nur 185,33 m³/d = 7,72 m³/h Wasser verdampft werden. Mit einer Trocknungseffektivität von 1 MW_{Wärme}/m³ Wasser besteht somit ein Wärmebedarf von 7,72 MW. Das sind 3 MW weniger als nach dem herkömmlichen Verfahren.

Die einzelnen Behandlungsstufen müssen daher wie folgt ausgelegt werden:

| Durchsatz hydraulisch/d / Durchsatz TRP/d Verdampfungsleistung | | | |
|---|---|---|---|
| Entwässerung | 1.080 m³/d | 104,4 t/d | |
| Eindampfung | 540 m³/d | | 15 m³/h |
| Trocknung | 240 t/d | | 7,72 m³/h |

Die erforderliche Entwässerungsleistung der Schneckenpressen erhöht sich ca. um 50 %. Hierbei handelt es sich um eine Niedrig-Technologie-Stufe mit nur geringen Auswirkungen auf die Invest- und Betriebskosten.

Die erforderliche Eindampfleistung (bestimmt nur durch die Verdampfungs- und Kondensationsleistung) erhöht sich nur um ca. 25 %. Das ist investiv und insbesondere seitens der Betriebskosten viel weniger als die ca. 40 % Einsparung der Trocknungsleistung. Der Hauptgrund hierfür ist, dass bei der Eindampfung mit Kondensation über 90 % der erforderlichen Wärme zurückgewonnen werden kann.

Ein weiterer großer Vorteil des erfindungsgemäßen Verfahrens besteht darin, dass die Aufkonzentrierung in der Eindampfung auch geringer durchgeführt werden kann, als nach dem obigem Beispiel. Je nach Ionenzusammensetzung der Flüssigfraktion nach der Entwässerung könnte es z.B. ab 4 % TRI im Eindampfkonzentrat zu äußerst problematischen Salzausfällungen kommen, die im schlimmsten Fall eine Eindampfungsleistung auf über 4 % TRI unmöglich machen würde.

Nach dem herkömmlichen Verfahren würde dann entsprechend mehr Eindampfkonzentrat anfallen (mit entsprechend höheren Lager- und Transportkosten). Nach dem erfindungsgemäßen Verfahren würde diese Mehrmenge schlicht in der Wasserfraktion der entwässerten Gärreste entsorgt werden durch eine entsprechend höhere Rezirkulationsmenge an Eindampfkonzentrat.

Bei diesem Verfahren nach Anspruch 2 fallen nach obigem Beispiel zusätzlich ca. 180 m³/d Flüssigfraktion mit ca. 3 % TRI an. Diese können wahlweise direkt in die Eindampfung rezykliert werden (wie nach obigem Beispiel) oder sie können auch der zweiten Entwässenmgsstufe zugeführt werden. Dann würden mit dem entwässerten Gärrest der zweiten Entwässerungsstufe deutlich höhere TRI Mengen entfernt werden als nach dem herkömmlichen Verfahren. Das bedeutet, dass dann deutlich weniger als 180 m³/d Eindampfkonzentrat rezykliert werden müsste. Welche Alternative gewählt wird oder optimal ist, hängt vom einzelnen großtechnischen Einsatzfall ab.

In Fällen in denen keine oder nur geringe Mengen an Input - Substraten mit nennenswerten Strukturanteilen zugeführt werden, entfällt eine erste Niedrig-Technologie Entwässerungsstufe mit Schneckenpressen. Dann ist nur der Einsatz einer Hochtechnologie - Entwässerungsstufe, beispielsweise mit Zentrifugen, sinnvoll. In einem solchen Fall ist auch die Implementierung des Verfahrens nach Anspruch 2 möglich, allerdings weniger sinnvoll, da in einem solchen Fall die Rückmischung/Verdünnung mit Eindampfkonzentrat viel aufwendiger und teurer wäre und mit einem hohen zusätzlichen Verbrauch an Flockungshilfsmitteln verbunden wäre.

In einem solchen Fall wird die Rückmischung vor der Entwässerung vorgeschlagen (Anspruch 3):

### Beispiel: Rückmischung vor der Entwässerung:

### Bisherige Verfahrensweise:

Es wird ausgegangen von 480 m³/d Rohgärreste nach der Vergärung mit einem TRP von 6 % und einem TRI von 0,5 %. Der Einfachheit halber wird davon ausgegangen, dass die Entwässerung den TRP vollständig aus dem Rohgärrest entfernt und diesen auf 24 % TRP entwässert. (In der betrieblichen Praxis erfolgt die Entfernung der restlichen TRP Fraktionen aus den Flüssigfraktionen der Entwässerung meistens mittels einer nachfolgenden Hochlastbiologie, das ist aber für die vorliegenden Zwecke und Erläuterungen unerheblich und wird daher vernachlässigt). Die Bilanz der Entwässerung ergibt somit:

| | |
|---|---|
| Rohgärrest: | 480 m³/d, 6 % TRP, 0,5 % TRI, 93,5 % Wasser |
| Entwässerter Gärrest: | 120 t/d, 24 % TRP, 0,5 % TRI, 75,5 % Wasser |
| Flüssigfraktion: | 360 m³/d, 0 % TRP, 0,5 % TRI, 99,5 % Wasser |

Bei der Eindampfung wird von einer Aufkonzentrierung auf 10 % TRI ausgegangen. Bei der Bilanzierung werden die NH₄/NH₃ Konzentrationen nicht beachtet, weil diese im Rahmen der Eindampfung üblicherweise mittels Strippung eliminiert werden. Die Bilanz der Eindampfung ergibt somit:

| | |
|---|---|
| Flüssigfraktion aus der Entwässerung: | 360 m³/d 0 % TRP, 0,5 % TRI, 99,5 % Wasser |
| Eindampfkonzentrat: | 18 m³/d, 0 % TRP, 10 % TRI, 90 % Wasser |
| Flüssigfraktion = Kondensat: | 342 m³/d, 0 % TRP, 0 % TRI, 100 % Wasser |

Beide Konzentratfraktionen zusammen ergeben somit 138 t/d mit 20,87 % TRP und 1,74 % TRI (= 22,61 % TR). Der Rest mit 77,39 % = 107,8 m³ ist Wasser.

Die Bilanz der Trocknung dieser Fraktion auf 90 % TR ergibt somit:

| | |
|---|---|
| Konzentrate: | 138 m³/d, 22,61 % TR, 77,39 % Wasser |
| Trockengranulat: | 34,67 t/d, 90 % TR, 10 % Wasser |
| Verdampftes Wasser: | 103,33 m³/d, 0 % TR |

Nach diesem herkömmlichen Verfahren müssen somit bei vollständiger Gärresttrocknung 103,33 m³/d = 4,31 m³/h Wasser verdampft werden. Mit einer Trocknungseffektivität von 1 MW_{Wärme}/m³ Wasser besteht somit ein Wärmebedarf von 4,31 MW.

Für die Einbringung der flüssigen Eindampfkonzentrate in die Trocknung muss mit Trockengranulat rückgemischt werden; das beinhaltet erhebliche finanzielle Mehraufwendungen.

Die einzelnen Behandlungsstufen müssen daher wie folgt ausgelegt werden:

| Durchsatz hydraulisch/d / Durchsatz TRP/d Verdampfungsleistung | | | |
|---|---|---|---|
| Entwässerung | 480 m³/d | 28,8 t/d | |
| Eindampfung | 360 m³/d | | 14,25 m³/h |
| Trocknung | 138 t/d | | 4,31 m³/h |

### Verfahren nach Anspruch 3

Die 480 m³/d Rohgärrest werden mit Konzentrat der Eindampfung vermischt und der Entwässerung zugeführt. Damit der gesamte TRI in der Wasserphase des entwässerten Gärrestes überführt wird, muss genau 90 m³/d Eindampfkonzentrat rezirkuliert werden; genau dann bleibt kein Eindampfkonzentrat übrig. Die Bilanz dieser Entwässerung ergibt somit:

| | | | | | |
|---|---|---|---|---|---|
| Rohgärrest + Eindampfkonz.: | 570 m³/d, | 5,053 % | TRP, 2 % TRI, | 92,95 % | Wasser |
| Entwässerter Gärrest: | 120 t/d, | 24 % | TRP, 2 % TRI, | 74 % | Wasser |
| Flüssigfraktion: | 450 m³/d, | 0 % | TRP, 2 % TRI, | 98 % | Wasser |

Bei der Eindampfung wird wiederum von einer Aufkonzentrierung auf 10 % TRI ausgegangen.

Die Bilanz der Eindampfung ergibt somit:

| | | | | |
|---|---|---|---|---|
| Flüssigfraktion aus der Entwässerung: | 450 m³/d, | 0 % TRP, | 2 % TRI, | 98 % Wasser |
| Eindampfkonzentrat: | 90 m³/d, | 0 % TRP, | 10 % TRI, | 90 % Wasser |
| Flüssigfraktion = Kondensat: | 360 m³/d, | 0 % TRP, | 0 % TRI, | 100 % Wasser |

Da die 90 m³/d Eindampfkonzentrat vollständig rezirkuliert werden müssen (siehe oben), damit der ganze TRI in der Wasserphase der entwässerten Gärreste landet, verbleibt keinerlei zu speicherndes und zu entsorgendes Eindampfkonzentrat.

Somit muss nur der entwässerte Gärrest auf 90 % TR getrocknet werden:

| | |
|---|---|
| Entwässerter Gärrest: | 120 m³/d, 26 % TR, 74 % Wasser |
| Trockengranulat: | 34,67 t/d, 90 % TR, 10 % Wasser |
| Verdampftes Wasser: | 85,33 m³/d, 0 % TR |

Nach diesem Verfahren nach der Erfindung müssen somit bei vollständiger Gärresttrocknung nur 85,33 m³/d = 3,65 m³/h Wasser verdampft werden. Mit einer Trocknungseffektivität von 1 MW_{Wärme}/m³ Wasser besteht somit ein Wärmebedarf von 3,65 MW. Das sind 0,75 MW weniger als nach dem herkömmlichen Verfahren.

Die einzelnen Behandlungsstufen müssen daher wie folgt ausgelegt werden:

| Durchsatz hvdraulisch/d / Durchsatz TRP/d Verdampfungsleistung | | | |
|---|---|---|---|
| Entwässerung | 570 m³/d | 28,8 t/d | |
| Eindampfung | 450 m³/d | | 15 m³/h |
| Trocknung | 120 t/d | | 3,65 m³/h |

Da die Entwässerungsleistung ungefähr im gleichen Maße vom Durchsatz Hydraulik und Durchsatz TR bestimmt wird, erhöht sie sich ca. um 10 %. Die erforderliche Eindampfleistung (bestimmt nur durch die Verdampfungs- und Kondensationsleistung) erhöht sich nur um ca. 5 %. Das ist investiv und seitens der Betriebskosten viel weniger als die ca. 18 % Einsparung der Trocknungsleistung.

Ein weiterer großer Vorteil des erfindungsgemäßen Verfahrens besteht darin, dass die Aufkonzentrierung in der Eindampfung auch geringer durchgeführt werden kann, als nach dem obigem Beispiel. Je nach Ionenzusammensetzung der Flüssigfraktion nach der Entwässerung könnte es z.B. ab 8 % TRI im Eindampfkonzentrat zu äußerst problematischen Salzausfällungen kommen, die im schlimmsten Fall eine Eindampfungsleistung auf über 8 % TRI unmöglich machen würde.

Nach dem herkömmlichen Verfahren würde dann entsprechend mehr Eindampfkonzentrat anfallen (mit entsprechend höheren Lager- und Transportkosten). Nach dem erfindungsgemäßen Verfahren würde diese Mehrmenge schlicht in der Wasserfraktion der entwässerten Gärreste durch eine entsprechend höhere Rezirkulationsmenge an Eindampfkonzentrat entsorgt werden.

Selbstverständlich kann das Verfahren nach Anspruch 3 auch im Rahmen einer ersten Nieder-Technologie Entwässerungsstufe implementiert werden.

Des Weiteren können beide Verfahren beliebig miteinander kombiniert werden.

Des Weiteren können beide Verfahren auch mit großen Vorteilen eingesetzt werden, wenn keine Gärresttrocknung vorgesehen ist: Dann entfällt im Rahmen der erforderlichen Speichervolumina die Zwischenspeicherung der Eindampfkonzentrate und auch die Verwertung (insbesondere der Transport) der Eindampfkonzentrate.

## Patentansprüche

1. Verfahren zum Betreiben einer Biogasanlage, bei dem der anfallende Rohgärrest unter Gewinnung eines flüssigen Eindampfkonzentrats entwässert und getrocknet wird, **gekennzeichnet durch** Rückmischen des flüssigen Eindampfkonzentrats zu dem Rohgärrest.

2. Verfahren nach Anspruch 1, **dadurch gekennzeichnet, dass** das Rückmischen des flüssigen Eindampfkonzentrats zu dem Rohgärrest nach dessen Entwässerung erfolgt.

3. Verfahren nach Anspruch 1, **dadurch gekennzeichnet, dass** das Rückmischen des flüssigen Eindampfkonzentrats zu dem Rohgärrest vor dessen Entwässerung erfolgt.

4. Vorrichtung zur Durchführung des Verfahrens nach einem der vorangehenden Ansprüche.
